Europäisches Patentamt

European Patent Office

Office européen des brevets

(11) Veröffentlichungsnummer: **0 303 187 B1**

## EUROPÄISCHE PATENTSCHRIFT

(45) Veröffentlichungstag der Patentschrift: **10.03.93**

(21) Anmeldenummer: **88112707.0**

(22) Anmeldetag: **04.08.88**

(51) Int. Cl.⁵: **B01F 17/00**, C11D 1/83, C11D 1/72, A61K 7/08, C07C 43/11

(54) **Wässrige Zubereitungen ionischer Tenside mit erhöhter Viskosität.**

(30) Priorität: **13.08.87 DE 3726911**

(43) Veröffentlichungstag der Anmeldung:
**15.02.89 Patentblatt 89/07**

(45) Bekanntmachung des Hinweises auf die Patenterteilung:
**10.03.93 Patentblatt 93/10**

(84) Benannte Vertragsstaaten:
**DE FR IT**

(56) Entgegenhaltungen:
**EP-A- 0 000 235        EP-A- 0 019 734**
**EP-A- 0 025 654        EP-A- 0 113 798**
**FR-A- 2 336 365        US-A- 4 206 070**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Hensen, Hermann, Dr.**
**Rathmacherweg 13**
**W-5657 Haan(DE)**
Erfinder: **Tesmann, Holger, Dr.**
**Vennstrasse 61**
**W-4000 Düsseldorf 12(DE)**
Erfinder: **Stoll, Gerhard, Dr.**
**Danziger Strasse 69**
**W-4052 Korschenbroich 1(DE)**
Erfinder: **Gruber, Bert, Dr.**
**Albert-Schlangen-Strasse 28**
**W-5012 Bedburg(DE)**
Erfinder: **Lange, Fritz, Dr.**
**Bühne 22**
**W-4300 Essen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind wäßrige Zubereitungen von ionischen Tensiden, die durch Zusatz bestimmter Polyolmonoether und gegebenenfalls zusätzlich anorganischer Elektrolytsalze in ihrer Viskosität erhöht sind.

Es ist bekannt, daß wässrige Lösungen mancher Tenside durch Zusatz von anorganischen Elektrolytsalzen in ihrer Viskosität angehoben werden können. Es ist auch bekannt, daß Fettsäurealkanolamide, z. B. Kokosfettsäuremonoethanolamid, Laurinsäuremonoethanolamid, Ölsäurediethanolamid und Kokosfettsäurediethanolamid auf viele Tensidsysteme eine verdickende und die Schaumstabilität unter Fettbelastung erhöhende Wirkung haben. Weiterhin ist bekannt, daß z. B. Polyethylenglycoldifettsäureester und viele wasserlösliche Polymere auch eine verdickende Wirkung auf wässrige Tensidlösungen haben.

Die genannten Verdickungsmittel haben jedoch viele Nachteile: So weisen die mit Polyethylenglycolfettsäurediester verdickten Lösungen oft eine unzureichende Viskositätsstabilität bei Lagerung auf, wasserlösliche Polymere sind oft schwer auflösbar und bewirken ein unerwünschtes, schleimiges Fließverhalten mit der Neigung zum "Fädenziehen", was in kosmetischen Zubereitungen sehr unerwünscht ist. Fettsäurealkanolamide sind als Abkömmlinge von z. B. Diethanolamin, welches in jüngster Zeit verdächtigt wird, an der Bildung von Nitrosaminen beteiligt zu sein, in kosmetischen Zubereitungen zunehmend unerwünscht, da ein geringer Gehalt an freiem Alkanolamin als Nebenprodukt nicht restlos ausgeschlossen werden kann.

Es bestand daher die Aufgabe, neue Verdickungsmittel für wässrige Tensidzubereitungen zu finden, welche die vorgenannten Nachteile nicht besitzen und auch keine Derivate von Aminen oder Alkanolaminen sind.

Aus US-A-4 206 070 waren Mischungen von Alkylglycerylethern, nichtionischen Tensiden und Alkylbenzolsulfonaten bekannt. FR-A-2 336 365 beschreibt Alkyl-oxyethyl-glycerylether und Zusammensetzungen, die diese Polyether mit anderen Tensidkomponenten enthalten.

Es wurde gefunden, daß sich die Viskosität wässriger Zubereitungen von ionischen Tensiden durch bestimmte Polyolmonoether und deren Ethoxylate und anorganische Elektrolytsalze ohne die vorgenannten Nachteile stark erhöhen läßt.

Gegenstand der Erfindung sind daher wässrige Zubereitungen ionischer Tenside mit erhöhter Viskosität, dadurch gekennzeichnet, daß diese

(A) 5 - 10 Gew.-% wasserlöslicher ionischer Tenside
(B) 1 - 5 Gew.-% eines oder mehrerer Polyolmonoether der allgemeinen Formel I

(I)    $R^1\text{-}(OC_2H_4)_x\text{-}O\text{-}A\text{-}(OH)_{n-1}$

in der $R^1$ eine, bevorzugt lineare, Alkylgruppe oder 2-Hydroxyalkylgruppe mit 12-18 C-Atomen, $x = 0$ oder eine Zahl von 1 - 6, $A\text{-}(OH)_{n-1}$ der Rest eines aliphatischen Polyols mit 2 - 6 C-Atomen und n Hydroxylgruppen und n eine Zahl von 2 - 6, aber nicht größer als die Zahl der C-Atome des Polyols ist, oder eines Anlagerungsproduktes von bis zu (6-x) Mol Ethylenoxid an einen solchen Polyolmonoether.

(C) 1 - 8 Gew.-% eines anorganischen Elektrolytsalzes, bevorzugt eines Chlorids oder Sulfats eines Alkalimetalls oder des Magnesiums und
(D) wenigstens 75 Gew.-% Wasser enthalten.

Als wasserlösliche ionische Tenside (A) können anionische, zwitterionische und kationische Tenside enthalten sein. Geeignete ionische Tenside zeichnen sich durch eine lipophile, bevorzugt lineare Alkyl- oder Alkenylgruppe mit 8 - 18 C-Atomen und eine, bevorzugt endständig daran gebundene, in Wasser dissoziierende ionische Gruppe aus. Die anionische Gruppe kann z. B. eine Sulfat-($-OSO_3^-$), Sulfonat-($-SO_3^-$), Phosphat-($-O\text{-}PO_3^{--}$), oder Carboxylat-($-COO^-$)-Gruppe sein, die kationische Gruppe kann z. B. eine quartäre Ammoniumgruppe (z. B.$-\overset{+}{N}(CH_3)_3$) sein, die zwitterionischen Gruppen können z. B. $-\overset{+}{N}(CH_3)_2\text{-}CH_2\text{-}COO^-$ oder $-\overset{+}{N}(CH_3)_2\text{-}CH_2\text{-}SO_3^-$ sein.

Geeignete anionische Tenside sind z. B. Alkylsulfate, Alkylpolyglycolethersulfate, Alkansulfonate, Sulfobernsteinsäuremonoalkylester- und -monopolyethoxyalkylester, Monoalkylphospate, Acylisethionate, Acyltauride, Seifen und Acylsarcoside. Geeignete kationische Tenside sind z. B. Alkyl-trimethylammonium-halogenide, Alkyl-dimethyl-benzylammonium-halogenide, Alkyl-pyridinium-halogenide, Alkylimidazolinium-halogenide. Geeignete zwitterionische Tenside sind z. B. N-Alkyl-N,N-dimethylglycin oder N-Acylamino-propyl-N,N-dimethylglycin. Als ionische Tenside können auch Gemische der genannten anionischen oder Gemische aus anionischen oder kationischen und zwitterionischen Tensiden verwendet werden.

Besonders bevorzugt sind wässrige Zubereitungen, in welchen das ionische Tensid ein Alkylsulfat oder Alkylethersulfat der allgemeinen Formel II

(II)     $R^2\text{-}O(C_mH_{2m}O)_y\text{-}SO_3^- \ M^+$

ist, worin $R^2$ eine bevorzugt lineare Alkylgruppe mit 12 - 18 C-Atomen und m = 2 oder 3, y = 0 oder eine Zahl von 1 - 12 und $M^+$ ein Alkali-, Ammonium-, Mono-, Di- oder Trialkanolammoniumion mit 2 oder 3 C-Atomen in der Alkanolgruppe oder ein 1/2 $Mg^{++}$-Ion darstellt. Beispiele für solche bevorzugten Aniontenside sind z. B. die Fettalkoholsulfate auf Basis von $C_{12}$-$C_{16}$-oder $C_{12}$-$C_{18}$-Fettalkoholschnitten in Form ihrer Ammonium- oder Triethanolammoniumsalze und die Fettalkoholethersulfate auf Basis von Anlagerunsprodukten von 2 - 4 Mol Ethylenoxid an $C_{12}$-$C_{14}$ oder $C_{12}$-$C_{16}$-Fettalkoholschnitte in Form ihrer Natrium-, Ammonium- oder Magnesiumsalze.

Die Polyolmonoether der allgemeinen Formel I können Derivate des Ethylenglycols, des Glycerins, des Erythrits, des Pentaerythrits, des Trimethylolpropans, des Sorbits oder von cyclischen aliphatischen Polyolen wie z. B. Cyclohexantriol oder Inosit sein. Solche Polyolether sind überwiegend literaturbekannt oder lassen sich nach bekannten Verfahren herstellen. Man kann z. B. die genannten Polyole durch Alkylierung mit Alkylhalogeniden oder Alkylschwefelsäurehalbestersalzen mit 12 - 18 C-Atomen in den Alkylgruppen oder auch mit Alkyl($C_{12-18}$)-polyethylenglycoletherschwefelsäurehalbestersalzen mit x = 1-5 Ethylenglycolethergruppen in die Polyol-monoether überführen. Beispiele für solche Veretherungen werden in Beispiel 1.1 bis 1.3 angegeben. Ethylenglycol- oder Propylenglycol-monoether, die durch Anlagerung von Ethylenoxid oder Propylenoxid an z.B. Fettalkohole mit 12 bis 18 C-Atomen zugänglich sind, und die neben freiem Fettalkohol und höheroxalkylierten Homologen nur geringe Anteile an Glycol-monoethern enthalten, fallen nicht in den Bereich der Polyolmonoether der Formel 1.

Zur Herstellung von 2-Hydroxyalkyl-monoethern werden die genannten Polyole mit 1.2-Epoxyalkanen mit 12-18 C-Atomen umgesetzt. Beispiele hierfür werden in Beispiel 1.7, 1.9 und 1.12-1.14 gegeben. Untergeordnete Beimengungen der entsprechenden Polyoldiether, wie sie produktionsbedingt in technischen Polyol-monoethern der genannten Art enthalten sein können, sind für die erfindungsgemäßen Zubereitungen nicht nachteilig.

Außer den genannte Polyolen der allgemeinen Formel I sind auch die Anlagerungsprodukte von bis zu 6 Mol Ethylenoxid an diese Polyolether als Komponente B in den erfindungsgemäßen Zubereitungen geeignet. Im Falle dieser Ethylenoxid-Anlagerungsprodukte sollte aber die Summe der angelagerten Ethoxygruppen und der gegebenenfalls durch die Gruppierung $(OC_2H_4)_x$ gegebenen Ethoxygruppen nicht größer als 6 sein, d. h. es sollten nicht mehr als (6-x) Mol Ethylenoxid angelagert werden. Obwohl Polyolmonoether mit z. B. 6 angelagerten Glycolethergruppen noch eine deutliche Verdickungswirkung zeigen, sind doch solche Polyolether stärker wirksam, die nicht mehr als (4-x) Mol Ethylenoxid angelagert enthalten.

Besonders bevorzugt sind erfindungsgemäße wässrige Zubereitungen ionischer Tenside, wenn als Komponente B ein Polyolmonoether der Formel 1 verwendet wird, in der $R^1$ eine $C_{12}$-$C_{18}$-Alkyl- oder 2-Hydroxy-($C_{12}$-$C_{18}$)-alkylgruppe, und A -$(OH)_{n-1}$ ein Glycerinrest oder ein Trimethylolpropanrest ist. Im Falle des Glycerinrestes ist A = $C_3H_5$ und n = 3, im Falle des Trimethylolpropanrestes ist A = $C_6H_{11}$ und n = 3. Diese Polyolmonoether und die Anlagerungsprodukte von bis zu 4 Mol Ethylenoxid an die Polyolmonoether zeichnen sich durch besonders gute Verdickungswirkungen aus.

Wenn die Konzentration der Komponente A in den erfindungsgemäßen wässrigen Zubereitungen relativ hoch ist, z. B. wenn diese zwischen 10 und 20 Gew.-% liegt, so kann in vielen Fällen bereits ohne Zusatz von Kochsalz eine gewisse Viskositätserhöhung durch Zusatz von 1 - 5 Gew.-% der Polyolmonoether der allgemeinen Formel I erreicht werden. Von besonders hohem anwendungstechnischen Interesse ist jedoch die Viskositätserhöhung von wässrigen Lösungen mit relativ geringem Gehalt an ionischen Tensiden. In diesen Fällen verbessert der Zusatz der Polyolether der allgemeinen Formel I in synergistischer Weise die Verdickbarkeit mit anorganischen Elektrolytsalzen, so daß nur eine kleine Menge des Polyolethers und eine geringe Menge der Elektrolytsalze zur Verdickung erforderlich ist. Bei Tensidlösungen, die durch Zusatz von Elektrolytsalzen praktisch nicht verdickt werden können, läßt sich nach Zusatz der Polyolmonoether trotzdem eine relativ hohe Viskosität einstellen.

Eine besonders bevorzugte Ausführungsform der vorliegenden Erfindung sind wässrige Zubereitungen ionischer Tenside, in welchen die Summe der Komponenten A + B + C (ionisches Tensid + Polyolmonoether + Elektrolytsalz) zwischen 10 und 25 Gew.-% liegt.

Als anwendungstechnisch besonders relevant erscheinen dabei Viskositäten, wie sie für Shampoos, Duschbadpräparate, flüssige Seifen und ähnliche Produkte erreicht werden müssen, damit diese Produkte bei der Anwendung nicht wasserdünn durch die Finger rinnen, sondern sich nach Aufgabe auf die Hand ohne Schwierigkeiten auf dem Körper verteilen lassen. Dies sind Viskositäten von mindestens etwa 1 Pa.s (20 °C), gemessen mit dem Höppler Kugelfallviskosimeter.

Als anorganische Elektrolytsalze eignen sich alle wasserlöslichen Alkali- Ammonium- und Erdalkalisalze, z. B. die Fluoride, Chloride, Bromide, Sulfate, Phosphate, Nitrate, soweit sie in einer Menge von wenigstens 1 Gew.-% bei 20 °C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid.

Die erfindungsgemäßen wässrigen Zubereitungen können darüberhinaus weitere Komponenten enthalten, die sie für den jeweiligen Anwendungszweck geeignet machen. Diese können z. B. nichtionogene Tenside, bevorzugt in kleineren Mengen bis ca. 50 Gew.-% der enthaltenen ionogenen Tenside sein. In untergeordneten Mengen können schließlich Duftstoffe, Farbstoffe, Trübungs- und Perlglanzmittel, antimikrobielle Stoffe, Konservierungsmittel, hautkosmetische Wirkstoffe, Pflanzenextrakte, Proteinhydrolysalze, Puffersubstanzen, Komplexbildner und andere bekannte Hilfs- und Zusatzstoffe enthalten sein, wie sie in Shampoos, Badezusätzen, Duschbadpräparaten, flüssigen Seifen, flüssigen Hautreinigungsmitteln, flüssigen Haarspülmitteln, flüssigen Wasch- und Geschirrspülmitteln sowie flüssigen Haushaltsreinigungsmitteln auf Basis von ionogenen Tensiden üblich sind.

Die folgenden Beispiele sollen den Gegenstand der Erfindung näher erläutern.

Beispiele

1. Herstellung von Polyolmonoethern

1.1 Herstellung von Glycerinmonoalkyl ($C_{12/14}$)-ether

45 kg (488,7 Mol) Glycerin wurden in einem Reaktionskessel mit 7,2 kg (90 Mol) Natronlauge (50 Gew.-%ig in Wasser) zusammengegeben und unter Stickstoffatmosphäre auf 150 °C erwärmt, wobei Wasser abdestillierte. Der Ansatz wurde noch 14 Stunden nachgerührt. Danach wurden 26,1 kg (90 Mol) Fettalkohol ($C_{12/14}$)-sulfat, Na-Salz (Nadeln) zugegeben und das Reaktionsgemisch auf 175 °C erwärmt. Nach 8 Stunden Reaktionszeit unter ständigem Rühren wurde der Ansatz auf 90 °C gekühlt und zweimal mit 20 kg Wasser gewaschen. Nach Abtrennung der organischen Phase wurde diese bei 90 °C unter vermindertem Druck vom Restwasser befreit. Das erhaltene Produkt war eine farblose, feste Masse mit einer Hydroxylzahl (nach DGF-Einheitsmethode C-V-17a) von 366,3.

1.2 Alkyl ($C_{12/14}$)-diethylenglycolether-glycerinmonoether

Nach dem Verfahren gemäß Beispiel 1.1 wurde aus Glycerin und einem Fettalkohol ($C_{12/14}$)-diethylenglycolethersulfat, Na-Salz ein Polyolmonoether hergestellt. Das Produkt hatte eine Hydroxylzahl von 291,7.

1.3 Alkyl ($C_{12/14}$)-triethylenglycolether-glycerinmonoether

Nach dem Verfahren gemäß Beispiel 1.1 wurde aus Glycerin und einem Fettalkohol ($C_{12/14}$)-triethylenglycolethersulfat, Na-Salz ein Polyolmonoether hergestellt. Das Produkt hatte eine Hydroxylzahl von 256,7.

1.4 Glycerinmonoalkyl ($C_{12/14}$)-ether + 2 Mol Ethylenoxid

600 g des Reaktionsproduktes nach 1.1 wurden mit 4,7 g Natriummethylat-Lösung (30 Gew.-%ig in Methanol) in einem Druckgefäß unter Stickstoffatmosphäre nach Entfernung des Methanols bei einer Temperatur von 100 °C bis 150 °C mit 172,5 g Ethylenoxid (Molverhältnis 1:2) umgesetzt, wobei der Druck bis 5 bar anstieg. Nach beendeter Reaktion (Druckabfall) wurde der Ansatz noch 45 Minuten bei vermindertem Druck von 0,05 bar auf 70 °C gehalten. Es wurde eine gelbe Flüssigkeit mit einer Hydroxylzahl von 293,7 erhalten.

1.5 Glycerinmonoalkyl ($C_{12/14}$)-ether + 4 Mol Ethylenoxid

Nach dem gleichen Verfahren wie unter 1.4 wurde ein Anlagerungsprodukt von 4 Mol Ethylenoxid hergestellt. Das Produkte hatte eine Hydroxylzahl von 233,8.

1.6 Glycerinmonoalkyl ($C_{12/14}$)-ether + 6 Mol Ethylenoxid

Nach dem Verfahren 1.4 wurde ein Anlagerungsprodukt von 6 Mol Ethylenoxid hergestellt. Das Produkt hatt eine Hydroxylzahl von 202,0.

1.7 Trimethylolpropan-mono (2-hydroxydodecyl)-ether

160,8 kg (1200 Mol) Trimethylolpropan wurden in einem Reaktionsgefäß bei 60 - 65 °C aufgeschmolzen. Nach Zugabe von 0,08 kg KOH (45 Gew.-%ig in Wasser) wurde der Ansatz bei 100 °C und 0,01 bar unter Rühren vom Wasser befreit. Dann wurden 78,9 kg (429 Mol) 1,2-Epoxydodecan zugegeben und der Ansatz unter Stickstoffatmosphäre und ständigem Rühren auf 160 °C erhitzt. Nach ca. 2 Stunden Reaktionszeit war der Gehalt an Epoxidsauerstoff unter 0,1 Gew.-% abgesunken. Nach Abkühlung auf 100 °C wurde das Produkt mit 65 g Milchsäure (90 %ig) neutralisiert. Das überschüssige Trimethylolpropan wurde bei vermindertem Druck von 0,01 bar bei einer Temperatur bis 200 °C abdestilliert. Es wurden 116 kg Monoether mit einer Hydroxylzahl von 495,5 erhalten.

4

1.8 Trimethylolpropan-mono-(2-hydroxydodecylether) + 2 Mol Ethylenoxid

Nach dem Verfahren gemäß 1.4 wurde ausgehend von dem Polyolether nach 1.7 ein Anlagerungsprodukt von 2 Mol Ethylenoxid hergestellt. Das Addukt hatte eine Hydroxylzahl von 406.

1.9 Trimethylolpropan-mono-(2-hydroxytetradexyl)-ether

Nach dem gleichen Verfahren wie unter 1.5 wurde unter Einsatz von 1,2-Epoxytetradecan der 2-Hydroxytetradexylether mit einer Hydroxylzahl von 457,4 erhalten.

1.10 Trimethylolpropan-mono-(2-hydroxytetradecylether) + 2 Mol Ethylenoxid

Nach dem Verfahren gemäß 1.4 wurden an den Polyether nach 1.9 2 Mol Ethylenoxid angelagert. Das Addukt hatte eine Hydroxylzahl von 369.

1.11 Trimethylolpropan-mono-(1-hydroxytetradecylether) + 4 Mol Ethylenoxid

Nach dem Verfahren gemäß 1.4 wurden an den Polyether nach 1.9 4 Mol Ethylenoxid angelagert. Das Addukt hatte eine Hydroxylzahl von 313.

1.12 Trimethylolpropan-mono-(2-hydroxyhexadecyl)-ether

Nach dem gleichen Verfahren wie unter 1.5 wurde unter Zusatz von 1,2-Epoxyhexadecan der 2-Hydroxyhexadecylether mit einer Hydroxylzahl von 424,7 erhalten.

1.13 Trimethylolpropanmono-(2-hydroxy-$C_{12-18}$-alkyl)-ether

Nach dem Verfahren gemäß Beispiel 1.7 wurde aus Trimethylolpropan und einem Gemisch aus 90 Gew.-% 1.2-Epoxydodecan, 6 Gew.-% 1.2-Epoxytetradecan, 3 Gew.-% 1.2-Epoxyhexadecan und 1 Gew.-% 1.2-Epoxyoctadecan ein Polyolether hergestellt. Das Produkt hatte eine Hydroxylzahl von 497.

1.14 Ethylenglycol-mono-(2-hydroxydodecyl)-ether

Analog zu 1.7 wurde unter Verwendung von Ethylenglycol und 1.2-Epoxydodecan der Mono-2-hydroxydodecylether hergestellt. Das Produkt hatte eine Hydroxylzahl von 429,0.

1.15 Ethylenglycolmono-(2-hydroxydodecyl)-ehter + 2 Mol Ethylenoxid

Analog dem Verfahren nach 1.4 wurden an das Reaktionsprodukt von 1.14 2 Mol Ethylenoxid angelagert. Das Addukt hatte eine Hydroxylzahl von 342,8.

1.16 Ethylenglycolmono-(2-hydroxydodecyl)-ether + 4 Mol Ethylenoxid

Analog dem Verfahren nach 1.4 wurden an das Reaktionsprodukt von 1.14 4 Mol Ethylenoxid angelagert. Das Addukt hatte eine Hydroxylzahl von 260,3.

2. Anwendungstechnische Prüfungen

2.1 Verdickung von Alkylethersulfat-Lösungen

Es wurden Lösungen mit 10 Gew.-% eines Fettalkohol $C_{12/14}$(70:30)Polyglycolether-(2 EO)-sulfat, Natriumsalzes (Texapon [R] NSO, Henkel KGaA) mit Zusätzen von 1 und 3 Gew.-% der verschiedenen Polyolmonoether hergestellt und durch Zusätze von Kochsalz in Mengen von 1 - 10 Gew.-% verdickt.

In der Tabelle I sind die Viskositäten bei 20 °C (gemessen mit dem Höppler-Kugelfall-Viskosimeter) in Pa.s angegeben:

EP 0 303 187 B1

## Tabelle 1

Viskosität in Pa.s (20 °C) nach Zusatz von NaCl

| Polyether gemäß Beispiel | Zusatz Gew.-% | ohne NaCl | 1 Gew.-% NaCl | 2 Gew.-% NaCl | 3 Gew.-% NaCl | 4 Gew.-% NaCl | 5 Gew.-% NaCl | 6 Gew.-% NaCl | 7 Gew.-% NaCl | 8 Gew.-% NaCl |
|---|---|---|---|---|---|---|---|---|---|---|
| | ohne | <1 | <1 | <1 | <1 | <1 | 4 | 16 | 36 | |
| 1.2 | 3 | <1 | | | 8 | | 25 | | | |
| 1.3 | 3 | <1 | | 4,4 | 18 | | | | | |
| 1.4 | 3 | <1 | <1 | 1,1 | 15 | 1,3 | <1 | | | |
| 1.5 | 3 | <1 | | <1 | 1,4 | 4,5 | 7 | 4,4 | <1 | |
| 1.6 | 3 | <1 | | | | <1 | 2,1 | 3,4 | 4,5 | 3,3 |
| 1.7 | 3 | <1 | 23 | 1,7 | <1 | | | | | |
| 1.8 | 3 | <1 | | 6 | | | | | | |
| 1.9 | 3 | <1 | 16 | <1 | | | | | | |
| 1.10 | 3 | <1 | | 13 | | | | | | |
| 1.11 | 3 | <1 | | | 7,9 | | | | | |
| 1.7 | 1 | <1 | <1 | 1 | 11 | 8,5 | 2 | <1 | | |

EP 0 303 187 B1

2.2 Verdickung von Tensidgemsichen und Sulfosuccinaten.

Es wurden Lösungen mit 10 Gew.-% Aktivsubstanz der folgenden Tenside bzw. Tensidmischungen hergestellt:

2.2.1 Fettalkohol C$_{12/14}$ (70:30)-polyglycol (2 EO)ethersulfat Natriumsalz 75 Gew.-% N-Kokosacyl (C$_{12-13}$)-aminopropyl-N,N-dimethyl-glycin 25 Gew.-%

## Tabelle I

### (Fortsetzung)

Viskosität in Pa.s (20 °C) nach Zusatz von NaCl

| Polyether gemäß Beispiel | Zusatz Gew.-% | ohne NaCl | 1 Gew.-% NaCl | 2 Gew.-% NaCl | 3 Gew.-% NaCl | 4 Gew.-% NaCl | 5 Gew.-% NaCl | 6 Gew.-% NaCl | 7 Gew.-% NaCl | 8 Gew.-% NaCl |
|---|---|---|---|---|---|---|---|---|---|---|
| 1.9 | 1 | <1 | <1 | 1 | 13 | 16 | 2,2 | <1 | | |
| 1.12 | 1 | <1 | | <1 | 8 | 28 | 17 | | | |
| 1.12 | 3 | <1 | 9* | 15* | <1* | | | | | |
| 1.13 | 1 | <1 | | | 8,2 | 20 | <1 | | | |
| 1.13 | 3 | <1 | 34 | 9,1 | | | | | | |
| 1.15 | 3 | <1 | | 1 | 5,2 | 3,5 | 1,1 | <1 | | |
| 1.16 | 3 | <1 | | | <1 | 1,2 | 2,7 | 2,8 | 2,1 | 1,4 |

* Lösungen sind getrübt

EP 0 303 187 B1

2.2.2 Fettalkohol $C_{12/14}$ (70:30) polyglycol (2 EO)ethersulfat 80 Gew.-% Eiweißhydrolysat-Kokosfettsäure-Kondensat.
Kaliumsalz    20 Gew.-%
2.2.3 Fettalkohol-$C_{12/14}$ (70:30)-polyglycolether-(3 EO)-sulfobernsteinsäuremonoester-Natriumsalz
mit Zusätzen von 1 bzw. 3 Gew.-% des Polyolethers gemäß Beispiel 1.13 in Wasser hergestellt und durch Zugabe von Kochsalz verdickt. Die Ergebnisse sind der Tabelle 11 zu entnehmen.

Tabelle II

|  | 2.2.1 | | 2.2.2 | | 2.2.3 | |
|---|---|---|---|---|---|---|
| Tensid/Tensidgemisch | 10 | 10 | 10 | 10 | 10 | 10 |
| Polyol, Beispiel 1.13 | - | 1 | - | 3 | - | 3 |
| Na Cl-Zusatz | 1 | 1,0 | 0,5 | 0,5 | 6,5 | 6,5 |
| Wasser | ad 100 | ad 100 | ad 100 | ad 100 | ad 100 | ad 100 |
| Viskosität (Pa.s) | 1 | 6,7 | 1 | 12,2 | 1 | 8,0 |

**Patentansprüche**

1.  Wässrige Zubereitungen ionischer Tenside mit erhöhter Viskosität, gekennzeichnet durch einen Gehalt von

    (A) 5 - 10 Gew.-% wasserlöslicher ionischer Tenside
    (B) 1 - 5 Gew.-% eines oder mehrerer Polyolmonoether der allgemeinen Formel

    (I)    $R^1 - (OC_2H_4)_x - O - A - (OH)_{n-1}$

    in der $R^1$ eine bevorzugt lineare Alkylgruppe oder 2-Hydroxyalkylgruppe mit 12 - 18 C-Atomen, $x = 0$ oder eine Zahl von 1 - 6, A -$(OH)_{n-1}$ der Rest eines aliphatischen Polyols mit 2 - 6 C-Atomen und n Hydroxylgruppen und n eine Zahl von 2 - 6, aber nicht größer als die Zahl der C-Atome des Polyols ist, oder eines Anlagerungsproduktes von bis zu (6-x) Mol Ethylenoxid an einen solchen Polyolmonoether,
    (C) 1 - 8 Gew.-% eines anorganischen Elektrolytsalzes, bevorzugt eines Chlorids oder Sulfats des Ammoniums eines Alkalimetalls oder des Magnesiums und
    (D) wenigstens 75 Gew.-% Wasser.

2.  Wässrige Zubereitungen nach Anspruch 1, dadurch gekennzeichnet, daß die Summe (A + B + C) zwischen 10 und 20 Gew.-% der Zusammensetzung ausmacht.

3.  Wässrige Zubereitungen nach Anspruch 1 - 2, dadurch gekennzeichnet, daß als Komponente B ein Polyolmonoether der Formel I, in der $R^1$ eine $C_{12-18}$-Alkyl- oder 2-Hydroxy-($C_{12}$-$C_{18}$)-alkylgruppe und A -$(OH)_{n-1}$ ein Glycerinrest oder ein Trimethylolpropanrest ist oder ein Anlagerungsprodukt von bis zu 4 Mol Ethylenoxid an einen solchen Polyolmonoether enthalten ist.

4.  Wässrige Zubereitungen nach Anspruch 1 - 3, dadurch gekennzeichnet, daß das ionische Tensid (A) ein Alkylsulfat oder Alkylethersulfat der allgemeinen Formel II

    $R^2 -O - (C_mH_{2m}O)_y - SO_3^- -M^+$

    ist, worin $R^2$ eine bevorzugt lineare Alkylgruppe mit 12 - 16 C-Atomen, $m = 2$ oder 3, $y = 0$ oder eine Zahl von 1 - 12 und $M^+$ ein Alkali-, Ammonium-, Mono-, Di- oder Trialkanolammoniumion mit 2 oder 3 C-Atomen in der Alkanolgruppe oder ein $1/2$ $Mg^{(++)}$-Ion darstellt.

**Claims**

1.  Aqueous preparations of ionic surfactants showing increased viscosity, characterized by a content of

8

(A) 5 to 10% by weight water-soluble ionic surfactants,
(B) 1 to 5% by weight of one or more polyol monoethers corresponding to the following general formula

$$R^1\text{-}(OC_2H_4)_x\text{-}O\text{-}A\text{-}(OH)_{n\text{-}1} \qquad (I)$$

in which
$R^1$ is a preferably linear alkyl group or 2-hydroxyalkyl group containing 12 to 18 carbon atoms, $x = 0$ or a number of 1 to 6, $A\text{-}(OH)_{n\text{-}1}$ is the residue of an aliphatic polyol containing 2 to 6 carbon atoms and n hydroxyl groups and n is a number of 2 to 6, but no greater than the number of carbon atoms of the polyol, or of an adduct of up to (6-x) mol ethylene oxide with such a polyol monoether,
(C) 1 to 8 % by weight of an inorganic electrolyte salt, preferably a chloride or sulfate of ammonium, an alkali metal or magnesium and
(D) at least 75% by weight water.

2. Aqueous preparations as claimed in claim 1, characterized in that the sum (A + B + C) makes up from 10 to 20% by weight of the composition.

3. Aqueous preparations as claimed in claims 1 or 2, characterized in that component B is a polyol monoether corresponding to formula I, in which $R^1$ is a $C_{12-18}$ alkyl or 2-hydroxy-$(C_{12-18})$-alkyl group and $A\text{-}(OH)_{n\text{-}1}$ is a glycerol residue or a trimethylol propane residue, or an adduct of up to 4 mol ethylene oxide with such a polyol monoether.

4. Aqueous preparations as claimed in claims 1 to 3, characterized in that the ionic surfactant (A) is an alkyl sulfate or alkyl ether sulfate corresponding to the following general formula

$$R^2\text{-}O\text{-}(C_mH_{2m}O)_y\text{-}SO_3^-\text{-}M^+ \qquad (II)$$

in which
$R^2$ is a preferably linear $C_{12-16}$ alkyl group, $m = 2$ or 3, $y = 0$ or a number of 1 to 12 and $M^+$ is an alkali metal, ammonium, mono-, di- or trialkanolammonium ion containing 2 or 3 carbon atoms in the alkanol group or a $1/2\ Mg^{(++)}$ ion.

## Revendications

1. Compositions aqueuses de tensioactifs ioniques à viscosité augmentée, caractérisées en ce que qu'elles contiennent:
    (A) de 5 à 10 % en poids de tensioactifs ioniques solubles dans l'eau,
    (B) de 1 à 15 % en poids d'un ou plusieurs monoéthers de polyols de formule générale I

    $$(I) \qquad R^1\ (OC_2H_4)_x\text{-}O\text{-}\ A\ \text{-}\ (OH)_{n\text{-}1}$$

    dans laquelle $R^1$ est un groupe alkyle ou 2-hydroxyalkyle de préférence linéaire ayant de 12 à 18 atomes de carbone, $x = 0$ ou un nombre allant de 1 à 6, $A\ \text{-}(OH)_{n\text{-}1}$ est le reste d'un polyol aliphatique ayant de 2 à 6 atomes de carbone, et comprenant n groupes hydroxy, et n est un nombre allant de 2 à 6, mais non supérieur au nombre des atomes de carbone du polyol, ou d'un produit de fixation par addition de jusqu'à (6-x) moles d'oxyde d'éthylène sur un tel monoéther de polyol.
    (C) de 1 à 8 % en poids d'un sel électrolytique minéral, de préférence d'un chlorure ou sulfate d'ammonium, d un métal alcalin ou du magnésium, et
    (D) au moins 75 % en poids d'eau.

2. Compositions aqueuses selon la revendication 1, caractérisées en ce que la somme (A + B + C) représente entre 10 et 20 % en poids de la composition.

3. Compositions aqueuses selon la revendication 1 ou 2, caractérisées en ce que, en tant que composant B, est contenu un monoéther de polyol de formule I, dans lequel $R^1$ est un groupe alkyle en $C_{12-18}$ ou 2-hydroxyalkyle en $C_{12-18}$, et $A\text{-}(OH)_{n\text{-}1}$ est un radical glycérol ou un radical triméthylolpropane, ou un

9

produit d'addition de jusqu'à 4 moles d'oxyde d'éthylène sur un tel monoéther de polyol.

4.  Compositions aqueuses selon l'une quelconque des revendications 1 à 3, caractérisées en ce que le tensioactif ionique (A) est un alkylsulfate ou alkyléthersulfate de formule générale II

$$R^2\text{-}O\text{-}(C_mH_{2m}O)_y\text{-}SO_3\text{-}M^+$$

dans laquelle $R^2$ représente un groupe alkyle de préférence linéaire ayant de 12 à 16 atomes de carbone, m = 2 ou 3, y = 0 ou un nombre allant de 1 à 12, et $M^+$ représente un ion alcalin, d'ammonium, de mono-, di- ou trialcanolammonium ayant 2 ou 3 atomes de carbone dans le groupe alcanol, ou un 1/2 ion $mg^{(++)}$.